(19) Europäisches Patentamt
European Patent Office
Office européen des brevets

(11) EP 1 603 600 B1

(12) **EUROPEAN PATENT SPECIFICATION**

(45) Date of publication and mention of the grant of the patent:
**02.01.2013 Bulletin 2013/01**

(21) Application number: **04709496.6**

(22) Date of filing: **09.02.2004**

(51) Int Cl.:
***A61L 15/26*** *(2006.01)* ***A61L 15/42*** *(2006.01)*
***A61L 15/44*** *(2006.01)* ***A61K 9/16*** *(2006.01)*
***A61K 9/70*** *(2006.01)*

(86) International application number:
**PCT/US2004/003755**

(87) International publication number:
**WO 2004/080499 (23.09.2004 Gazette 2004/39)**

(54) **POLYMER COMPOSITIONS WITH BIOACTIVE SILVER, COPPER OR ZINC COMPOUNDS, MEDICAL ARTICLES, AND PROCESSES**

POLYMERZUSAMMENSETZUNGEN ENTHALTEND BIOAKTIVE SILBER-, KUPFER- ODER ZINK-VERBINDUNGEN, MEDIZINISCHE ARTIKEL UND PROZESSE

COMPOSITIONS POLYMERES CONTENANT DES COMPOSES BIOACTIFS D'ARGENT, DE CUIVRE OU DE ZINC, ARTICLES MEDICAUX ET PROCEDES ASSOCIES

(84) Designated Contracting States:
**AT BE BG CH CY CZ DE DK EE ES FI FR GB GR HU IE IT LI LU MC NL PT RO SE SI SK TR**

(30) Priority: **12.03.2003 US 387051**
**05.12.2003 US 728577**

(43) Date of publication of application:
**14.12.2005 Bulletin 2005/50**

(73) Proprietor: **3M Innovative Properties Company**
**St. Paul, MN 55133-3427 (US)**

(72) Inventors:
- **BURTON, Scott, A.**
**Saint Paul, MN 55133-3427 (US)**
- **HYDE, Patrick, D.**
**Saint Paul, MN 55133-3427 (US)**

(74) Representative: **Vossius & Partner**
**Siebertstrasse 4**
**81675 München (DE)**

(56) References cited:
**DE-A- 19 958 697** **US-A- 3 685 993**
**US-A- 4 340 043** **US-A- 4 528 321**
**US-A- 6 039 940**

- **DATABASE WPI Section Ch, Week 199140 Derwent Publications Ltd., London, GB; Class A96, AN 1991-291352 XP002289245 & JP 03 193047 A (COTECH KK) 22 August 1991 (1991-08-22)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 603 600 B1

## Description

### BACKGROUND

Polymer compositions that include bioactive agents (e.g., antimicrobial agents) are used for a variety of applications, particularly medical applications such as wound dressings and wound packing materials. Conventional antimicrobial agents include ionizable silver compounds (e.g., silver salts such as silver nitrate); however, they are typically not light stable and leave a stain on skin with which they come into contact. Thus, stable antimicrobial polymer compositions are desired.

### SUMMARY

The present invention is directed to polymer compositions that include a bioactive agent (e.g., an antimicrobial agent). Such compositions are useful in medical articles, particularly wound dressings, wound packing materials, topical creams, and topical lotions, although a wide variety of other products can incorporate the polymer compositions. The bioactive agent is typically a silver compound, a copper compound, a zinc compound, or combinations thereof. Of these, it is more typically a silver compound. Such compositions are preferably stable. By this it is meant that the compositions are stable to at least one of the following types of radiation: visible light, ultraviolet light, electron beam, and gamma ray sterilization.

In one embodiment, the present invention provides a polymer composition preparable by a method that includes: combining components that include: an organic polymer; an inverse emulsion containing absorbent hydrophilic microparticles, which when in a substantially nonhydrated form have an average particle size of 10 microns or less, and wherein the microparticles include an amine-containing organic polymer selected from the group consisting of poly (quaternary amines), polylactams, polyamides, and combinations thereof; a bioactive agent selected from the group consisting of a silver compound, a copper compound, a zinc compound, and combinations thereof, wherein the silver compound has a solubility in water of at least 0.1 gram per liter in water; and an optional foaming agent; wherein the components are combined in a manner to produce a polymer composition wherein at least a portion of the bioactive agent is incorporated within the microparticles.

In another embodiment, the present invention provides a polymer composition that includes a hydrophilic amine-containing polymer having a weight average molecular weight of at least 1000 selected from the group consisting of poly(quaternary amines), polylactams, polyamides, and combinations thereof, and a bioactive agent dispersed therein, wherein the bioactive agent is a silver compound that has a solubility in water of at least 0.1 gram per liter in water.

Preferably, the polymer composition optionally includes a second organic polymer, thereby forming a mixture or blend of polymers. The second organic polymer is preferably a hydrophobic material. In one embodiment, the hydrophobic material forms a continuous matrix and the hydrophilic amine-containing polymer forms a discontinuous phase (e.g., microparticles). In another embodiment, the hydrophobic material forms a discontinuous phase and the hydrophilic amine-containing polymer forms a continuous matrix. In still another embodiment, the hydrophobic material forms a bicontinuous or co-continuous phase with the hydrophilic amine-containing polymer.

The present invention also provides medical articles that include the polymer compositions. The medical articles can be any of a wide variety of products, but preferably are wound dressings, wound packing materials, topical creams, or topical lotions.

In certain embodiments, the present invention provides a wound dressing that includes an apertured liquid permeable substrate and a nonadherent composition of the present invention.

The present invention also provides methods of making and using the polymer compositions.

As used herein, "a," "an," "the," "at least one," and "one or more" are used interchangeably. Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments.

### DETAILED DESCRIPTION OF ILLUSTRATIVE EMBODIMENTS OF THE INVENTION

The present invention provides polymer compositions that include an amine-containing polymer, an optional second organic polymer, and a bioactive agent distributed therein. The polymer composition can be in a wide variety of forms, such as an extruded film (e.g., having a thickness of 0.5 millimeter (mm) to 10 mm), a coating, a foam, particles, a hydrocolloid (i.e., a material that contains particles dispersed in a second phase, typically, hydrophilic particles dispersed in a lipophilic phase), a gel, a lotion, a cream, a molded article, etc.

In certain embodiments, the hydrophilic amine-containing polymer is selected from the group consisting of poly (quaternary amines), polylactams, polyamides, and combinations thereof. In certain embodiments, the hydrophilic amine-containing polymer is in the form of microparticles. The second organic polymer in certain embodiments forms a continuous matrix, and in certain embodiments is a hydrophobic material.

The bioactive agent is typically selected from the group consisting of a silver compound, a copper compound, a zinc compound, and combinations thereof. Of these, it is more typically a silver compound. In certain embodiments, the polymer composition is preparable from an organic polymer and an inverse emulsion that includes absorbent hydrophilic microparticles.

Such compositions are preferably stable. By this it is meant that the compositions are stable to at least one of the following types of radiation: visible light, ultraviolet light, electron beam, and gamma ray sterilization. Such compositions are useful in medical articles, particularly wound dressings, wound packing materials, topical creams, and topical lotions, although a wide variety of other products can incorporate the polymer compositions. The wound dressings can be used in their hydrated or swollen forms if desired.

In certain embodiments, the compositions of the present invention are nonadherent, although it should be understood that an adhesive (e.g., a pressure sensitive adhesive) could be added to an article that includes the composition. As used herein, the compositions of the present invention coated on a substrate display a 180° peel strength of less than 1 N/cm from steel according the to test procedure described in the Examples Section. Preferably, the compositions of the present invention do not adhere significantly to wound tissue such that they do not cause pain and/or destruction of the wound tissue upon removal.

AMINE-CONTAINING POLYMER

The amine-containing organic polymer is selected from the group consisting of poly(quaternary amines), polylactams, polyamides, and combinations thereof (including blends, mixtures, or copolymers thereof). Preferably, these are hydrophilic polymers (i.e., having an affinity for, absorbing, wetting smoothly with, tendency to combine with, or capable of dissolving in water).

Preferably, the amine-containing polymer has a weight average molecular weight of at least 1000. Examples include, but are not limited to, polyvinyl pyrrolidone, polyvinyl caprolactam, poly-N-vinylacetamide, poly-N-vinyl formamide, polyacrylamide, and the like.

Preferably, the amine-containing organic polymer includes a quaternary amine, and more preferably, the amine-containing polymer is a quaternary ammonium salt of an organic polymer. Such polymers are preferred typically because they can stabilize the bioactive compounds (particularly, silver compounds) effectively, they provide good release of the bioactive compounds, and they are absorbing of water or bodily fluids (e.g., wound exudate). Examples include, but are not limited to, polymerization products of cationic vinyl monomers as disclosed in EP 0 489 967 A1, and inherently antimicrobial quaternary amine polymers as described in U.S. Pat. No. 6,039,940.

Other suitable amine-containing polymers can be prepared from a quaternary ammonium monomer which is a salt having an group an a monoethylenically unsaturated group. For certain embodiments, the quaternary ammonium monomer has the following general Formula (I):

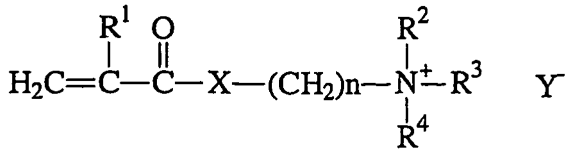

Formula (I)

wherein: n is 2 to 10, preferably 2 to 3; $R^1$ is H or $CH_3$; $R^2$, $R^3$, and $R^4$ are each independently linear or branched organic groups, preferably having 1 to 16 carbon atoms (on average); X is O or NH; and $Y^-$ is an acceptable anionic counterion to the $N^+$ of the quaternary ammonium group (e.g., one that does not adversely affect the polymerization of the monomers or antimicrobial activity of an added antimicrobial agent).

Preferably, $R^2$, $R^3$, and $R^4$ are each independently alkyl, aryl, alkaryl, or aralkyl groups. Alkyl groups are preferably lower alkyl, having 1 to 16 carbon atoms (on average) with methyl and ethyl groups being particularly preferred. Aryl is preferably phenyl but can be any suitable aromatic moiety such as those selected from the group consisting of phenyl, thiophenyl, naphthyl, biphenyl, pyridyl, pyrimidinyl, pyrazyl, pyridazinyl, furyl, thienyl, pyrryl, quinolinyl, bipyridyl, and the like. Representative of an aralkyl grouping is benzyl and representative of an alkaryl grouping is tolyl. X is

preferably O. Representative counterions ($Y^-$) are $Cl^-$, $Br^-$, HSO4-, $CH_3CH_2OSO_3^-$, and $CH_3OSO_3^-$, with the chloride salts being particularly preferred. Alkyl groups can be straight or branched chain and alkyl and aryl groups can be substituted by non-interfering substituents that do not obstruct with the functionality of the polymers.

Useful copolymerizable quaternary ammonium monomers include, but are not limited to, those selected from 2-(meth)acryloxyethyl trialkyl ammonium halides and sulfates, and mixtures thereof. Examples of such compounds include, but are not limited to, 2-(meth)acryloxyethyl trimethyl ammonium chloride, $CH_2$=C(H or $CH_3$)$CO_2CH_2CH_2N(CH_3)_3Cl$; 2-(meth)acryloxyethyl trimethyl ammonium methyl sulfate, $CH_2$=C(H or $CH_3$)$CO_2CH_2CH_2N(CH_3)_3OSO_2OCH_3$; 2-(meth)acryloxyethyl methyl diethyl ammonium methyl sulfate, $CH_2$=C(H or $CH_3$)$CO_2CH_2CH_2N(CH_3)(C_2H_5)_2OSO_2OCH_3$; 2-(meth)acryloxyethyl dimethyl benzyl ammonium chloride, $CH_2$=C(H or $CH_3$)$CO_2CH_2CH_2N(CH_3)_2(C_6H_5CH_2)Cl$ (all of the preceding monomers available from Ciba Specialty Chemicals, Woodbridge, NJ); 2-(methylacryloxy)ethyl dimethyl hexadecyl ammonium bromide, $CH_2=C(CH_3)CO_2CH_2CH_2N(CH_3)_2(C_{16}H_{33})Br$ (described in U.S. Pat. No. 5,437,932 (Ali et al.)); and the like. Various combinations of these monomers can be used if desired. Due to their availability, effectiveness in reinforcing (meth)acrylate polymers, and their antimicrobial activity, particularly preferred quaternary ammonium monomers are 2-acryloxyethyl trimethyl ammonium methyl chloride and 2-acryloxyethyl methyl diethyl ammonium methyl chloride. Such monomers are typically hydrophilic. Various combinations of other monoethylenically unsaturated monomers that are reinforcing monomers can be used in the polymers of the present invention. Such reinforcing monomers include, but are not limited to, acrylic acid, methacrylic acid, ethylene vinyl acetate, and N,N-dimethylacrylamide.

As an alternative approach to providing polymers that contain a quaternary ammonium functional unit, it is possible to start with an amine monomer and form the quaternary ammonium unit following polymerization. For certain embodiments, the amine monomers have the following general Formula (II):

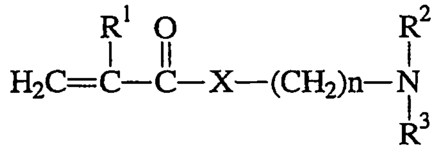

Formula (II)

wherein n, $R^1$, $R^2$, $R^3$, and X are the same as defined for Formula (I).

For certain embodiments, the amine-containing organic polymer (which is preferably in the form of microparticles) is absorbent (e.g., capable of absorbing water or bodily fluids). More preferably, the amine-containing organic polymer (which is preferably in the form of microparticles) is superabsorbent. In this context, "superabsorbent" means that the material will absorb at least 100% of its weight.

For certain embodiments, the amine-containing polymer is in the form of particles. If the amine-containing polymer is in the form of particles, it is typically in the form of microparticles. Preferably, the microparticles, when in a substantially nonhydrated form, have an average particle size of 10 microns or less, and more preferably, 1 micron or less. Typically and preferably, the microparticles have an average particle size of 0.5 micron or more when in a substantially nonhydrated form.

Preferred microparticles are as described in EP 172 724 A2 and EP 126 528 A2 made by reverse phase polymerization and have a dry particle size below 4 microns. The microparticles can be in an emulsion, such as an inverse emulsion that includes absorbent hydrophilic microparticles.

One type of inverse emulsion can be defined as a continuous hydrophobic liquid phase (e.g., mineral oil) and hydrophilic polymer particles dispersed within the hydrophobic liquid phase. Suitable examples of such materials are described in EP 0 126 528 A2. Such a material is commercially available under the trade designation SALCARE from Ciba Specialty Chemicals (High Point, NC). Suitable examples include SALCARE 95 and 96 which include a cationic homopolymer of the methyl chloride quaternary salt of 2-(dimethylamino)ethyl methacrylate (CAS No. 26161-33-1).

Other amine-containing polymers can be made from amine-containing monomers as described below and in EP 0 489 967 A1 and U.S. Pat. No. 6,039,940.

Monomers can be polymerized using techniques such as solution polymerization, emulsion polymerization, bulk polymerization, suspension polymerization, and the like. In particular, emulsion polymerization and suspension polymerization are preferable because the molecular weight of the polymer becomes high; solution polymerization is preferable because the molecular weight distribution is comparatively narrow; and bulk polymerization is favorable because no solvent is used.

In such polymerizations, initiators can be used to generate free-radicals upon the application of activating energy such as those conventionally used in the polymerization of ethylenically unsaturated monomers. Included among useful free-radical initiators are the thermally activated initiators such as organic peroxides, organic hydroperoxides, and azo-compounds. Representative examples of such initiators include, but are not limited to, benzoyl peroxide, tertiary-butyl perbenzoate, diisopropyl peroxydicarbonate, cumene hydroperoxide, azobis(isobutyronitrile), and the like. Generally, the thermal initiators are typically used in amounts from 0.01 to 5 percent by weight of monomer.

The polymerization of the polymer may also be initiated by photoinitiators. Such photochemically activated initiators are well known and have been described in the polymerization art; e.g., Chapter II of "Photochemistry" by Calvert and Pitts, John Wiley and Sons (1966) and in Progress in Organic Coatings, 13, 123-150 (1985). Representative examples of such initiators include benzoin, benzoin methyl ether, benzoin isopropyl ether, benzoin isobutyl ether, and 2-hydroxy-2-methyl-1-phenyl-1-propane, benzildimethylketal and benzildiethylketal, 2-hydroxy-1-(4-(2-hydroxyethoxy) phenyl)-2-methyl-1-propanone. A presently preferred photoinitiator is 2-hydroxy-1-(4-(2-hydroxyethoxy)phenyl)-2-methyl-1-propanone. Generally, photoinitiators are used in amounts from 0.01 to 5 percent by weight of monomer.

The polymerization of the polymer may also be initiated by electromagnetic radiation such as electron beams and the gamma-rays of cobalt 60, and the like. The irradiation dose is typically between 1 and 100 kGy.

The polymer may be crosslinked by adding a crosslinking compound or through electron beam or gamma radiation. A crosslinking compound can be a multi-ethylenically unsaturated compound wherein the ethylenic groups are vinyl groups, allyl groups, and/or methallyl groups bonded to nitrogen or oxygen atoms. Exemplary compounds include divinyl, diallyl or dimethallyl esters (e.g., divinyl succinate, divinyl adipate, divinyl maleate, divinyl oxalate, divinyl malonate, divinyl glutarate, diallyl itaconate, diallyl maleate, diallyl fumarate, diallyl diglycolate, diallyl oxalate, diallyl adipate, diallyl succinate, diallyl azelate, diallyl malonate, diallyl glutarate, dimethallyl maleate, dimethallyl oxalate, dimethallyl malonate, dimethallyl succinate, dimethallyl glutarate, and dimethallyl adipate), divinyl, diallyl or dimethallyl ethers (e.g., diethyleneglycol divinyl ether, butanediol divinyl ether, ethylene glycol divinyl ether, ethylene glycol diallyl ether, diethylene glycol diallyl ether, butane diol diallyl ether, ethylene glycol dimethallyl ether, diethylene glycol dimethallyl ether, and butane diol dimethallyl ether), divinyl, diallyl or dimethallyl amides including bis(N-vinyl lactams), (e.g., 3,3'-ethylidene bis(N-vinyl-2-pyrrolidone)), and divinyl, diallyl or dimethallyl ureas.

Amine-containing polymers can be used in a variety of combinations. The total amount of amine-containing polymer(s) (e.g., microparticles) is preferably at least 1 percent by weight (wt-%), and more preferably, at least 5 wt-%, based on the total weight of the polymer composition. The total amount of amine-containing polymer(s) (e.g., microparticles) is preferably at most 60 percent by weight (wt-%), based on the total weight of the polymer composition.

BIOACTIVE AGENT

The polymer compositions of the present invention typically include a bioactive agent selected from the group consisting of a silver compound, a copper compound, a zinc compound, and combinations thereof. The silver, copper, and zinc compounds are typically in the form of salts. Preferably, the bioactive agent is a silver compound.

At least, the silver compound has a solubility in water of at least 0.1 gram per liter, and more preferably, the silver, copper, and zinc compounds each have a solubility in water of at least 0.1 gram per liter. Sufficient solubility is desirable such that the compounds are dissolved into the hydrophilic amine-containing polymer phase, although for certain embodiments silver, copper, and zinc compounds having lower solubilities can be tolerated as long as they are leachable. However, silver halide salts are undesirable because they are too insoluble.

Such compounds are typically antimicrobial, although they can also demonstrate other activities, such as antifungal activity. Examples include, but are not limited to, silver oxide, silver nitrate, silver acetate, silver lactate, silver sulfate, copper chloride, copper oxide, copper nitrate, copper acetate, copper lactate, copper sulfate, zinc chloride, zinc oxide, zinc nitrate, zinc acetate, zinc lactate, and zinc sulfate.

One or more bioactive agents of this type can be used. Herein, these are considered the primary bioactive agents. Optionally, one or more secondary bioactive agents (e.g., antimicrobial agents, antibiotics) can be used in combination with these primary bioactive agents. Preferred compositions have more than one bioactive agent.

The bioactive agent can be present in the polymer composition in an amount to produce a desired effect (e.g., antimicrobial effect). Preferably, the bioactive agent is present in an amount such that the polymer composition is stable. In this context, "stable" means the composition does not turn black over a typical exposure time in the presence of at least one of the following types of radiation: visible light, ultraviolet light, electron beam, and gamma ray sterilization.

A preferred molar ratio of the bioactive agent (e.g., silver compound) to amine-containing monomers (for the embodiments that prepare the polymer *in situ*) is at least 1 mole bioactive agent to 500 moles amine-containing monomer. Although there is essentially no upper limit, a preferred molar ratio is no more than 1 mole bioactive agent to 40 moles amine-containing monomer.

A preferred weight ratio of the bioactive agent (e.g., silver compound) to amine-containing polymers (for the embodiments that mix the bioactive agent with a previously prepared polymer) is at least 0.1 weight percent (more

preferably at least 1 weight percent) bioactive agent based on the total weight of the amine-containing polymer. Although there is essentially no upper limit, a preferred weight ratio is no more than 3 weight percent (more preferably no more than 2 weight percent) bioactive agent based on the total weight of the amine-containing polymer.

SECONDARY POLYMER

The polymer compositions can include one or more secondary organic polymers in addition to one or more amine-containing polymers. These can be liquids or solids at room temperature. This secondary polymer can by hydrophobic or hydrophilic, although preferably it is hydrophobic (i.e., antagonistic to, shedding, tending not to combine with, or incapable of dissolving in water).

Examples of hydrophilic materials include, but are not limited to, polysaccharides, polyethers, polyurethanes, polyacrylates, polyesters, and alginates. Examples of hydrophobic materials include, but are not limited to, polyisobutylene, polyethylene-propylene rubber, polyethylene-propylene diene-modified (EPDM) rubber, polyisoprene, styrene-isoprene-styrene, styrene-butadiene-styrene, styrene-ethylene-propylene-styrene, and styrene-ethylene-butylene-styrene. Hydrophobic materials are particularly desirable for nonadherent compositions and articles. Particularly preferred hydrophobic materials include styrene-isoprene-styrene and styrene-ethylene-butylene-styrene, and even more preferred materials include styrene-isoprene-styrene.

The secondary polymer can be in the form of a continuous matrix (i.e., phase) or a discontinuous matrix (e.g., in the form of particles). It can form a bi-continuous or co-continuous phase with the amine-containing polymer. The secondary organic polymer can be elastomeric, thermoplastic, or both.

Elastomeric polymers useful as optional secondary polymers in the invention are typically materials that form one phase at 21°C, have a glass transition temperature less than 0°C, and exhibit elastomeric properties. The elastomeric polymers include, but are not limited to, polyisoprenes, styrene-diene block copolymers, natural rubber, polyurethanes, polyether-block-amides, poly-alpha-olefins, (C1-C20) acrylic esters of meth(acrylic) acid, ethylene-octene copolymers, and combinations thereof. Elastomeric materials useful in the present invention include, for example, natural rubbers such as CV-60 (a controlled viscosity grade natural rubber having Mooney viscosity of 60 +/- 5 ML, 1+4 at 100°C, available as an International commodity); butyl rubbers, such as Exxon Butyl 268 available from Exxon Chemical Co., Houston, Texas; synthetic poly-isoprenes such as CARIFLEX IR309, available from Kraton Polymers, Houston, Texas, and NATSYN 2210, available from Goodyear Tire and Rubber Co., Akron, Ohio; ethylene-propylenes; polybutadienes; polyisobutylenes such as VISTANEX MM L-80, available from Exxon Mobil Chemical Co.; and styrene-butadiene random copolymer rubbers such as AMERIPOL 1011A, available from BF Goodrich of Akron, Ohio.

Thermoplastic polymers useful as optional secondary polymers in the invention include, for example, polyolefins such as isotactic polypropylene; low density or linear low density polyethylene; medium density polyethylene; high density polyethylene; polybutylene; polyolefin copolymers or terpolymers, such as ethylene/propylene copolymer and blends thereof; ethylene-vinyl acetate copolymers such as ELVAX 260, available from E. I. DuPont de Nemours & Co., Wilmington, Delaware; ethylene acrylic acid copolymers; ethylene methacrylic acid copolymers such as SURLYN 1702, available from E. I. DuPont de Nemours & Co.; polymethylmethacrylate; polystyrene; ethylene vinyl alcohol; polyester; amorphous polyester; polyamides; fluorinated thermoplastics such a polyvinylidene fluoride; polytetrafluoroethylene; fluorinated ethylene/propylene copolymers; halogenated thermoplastics such as a chlorinated polyethylene; and combinations thereof. Other exemplary thermoplastic polymers are disclosed in International Publication No. WO 97/23577.

Thermoplastic elastomeric polymers useful as optional secondary polymers in the invention are typically materials that form at least two phases at 21°C, flow at a temperature greater than 50°C and exhibit elastomeric properties. Thermoplastic elastomeric materials useful in the present invention include, for example, linear, radial, star and tapered styrene-isoprene block copolymers such as KRATON D1107P, available from Kraton Polymers, and EUROPRENE SOL TE 9110, available from EniChem Elastomers Americas, Inc. Houston, Texas, linear styrene-(ethylene/butylene) block copolymers such as KRATON G1657 available from Kraton Polymers, linear styrene-(ethylene/propylene) block copolymers such as KRATON G1657X available from Kraton Polymers, styrene-isoprene-styrene block copolymers such as KRATON D1119P available from Kraton Polymers, linear, radial, and star styrene-butadiene block copolymers such as KRATON D1118X, available from Kraton Polymers, and EUROPRENE SOL TE 6205 available from EniChem Elastomers Americas, Inc., polyetheresters such as HYTREL G3548, available from E. I. DuPont de Nemours & Co., and poly-alpha-olefin based thermoplastic elastomeric materials such as those represented by the formula $-(CH_2-CHR)$ where R is an alkyl group containing 2 to 10 carbon atoms and poly-alpha-olefins based on metallocene catalysis such as ENGAGE EG8200, an ethylene/l-octen copolymer available from DuPont Dow Elastomers Co., Wilmington, Delaware. Other exemplary thermoplastic elastomers are disclosed in International Publication No. WO 96/25469.

Various combinations of secondary organic polymers in various amounts can be used to produce desired effects. This can be readily determined by one of skill in the art based on the teachings herein.

OPTIONAL ADDITIVES

The polymer compositions of the present invention can include a wide variety of optional additives. Examples include, but are not limited to, secondary bioactive agents, secondary absorbent particles, foaming agents, swelling agents, fillers, pigments, dyes, plasticizers (for example, mineral oil and petrolatum), tackifiers, crosslinking agents, stabilizers, compatibilizers, extruding aids, chain transfer agents, and combinations thereof.

In addition to the bioactive agents described above (e.g., silver, copper, and zinc compounds), other (secondary) bioactive agents can be incorporated into the polymer compositions of the present invention. Examples include, but are not limited to, antimicrobial agents such as parachlorometaxylenol, chlorhexidine and salts thereof, iodine, and iodophores, and antibiotics such as neomycin, bacitracin, and polymyxin B. Preferred compositions have more than one bioactive agent.

In certain embodiments, polymer compositions of the present invention can include secondary absorbent particles. Such secondary particles have an average particle size of greater than 10 microns when in a substantially nonhydrated form. Preferably, such particles are superabsorbent. Examples include, but are not limited to, those described in U.S. Pat. No. 5,369,155.

In certain embodiments, polymer compositions of the present invention can include a foaming agent. The foaming agent can be a chemical foaming agent or a physical foaming agent such as those disclosed in International Publication No. WO 00/74916 and in U.S. Pat. Nos. 6,103,152, 5,476,712, and 6,284,362. Of these foaming agents, the thermally expandable microspheres described in U.S. Pat. No. 6,103,152 are desirable for certain embodiments. Use of such thermally expandable microspheres in absorbent articles is further described in Applicants' Assignee's Copending Application Serial No. 10/387,263, filed March 12, 2003, published as US 2006 173087.

In certain embodiments, polymer compositions of the present invention can include a swelling agent, preferably a nonvolatile swelling agent. Examples of swelling agents include, but are not limited to, polyols, monosaccharides, ether alcohols, and combinations thereof. Specific examples are disclosed in U.S. Pat. No. 5,270,358.

In certain embodiments, polymer compositions of the present invention can include fillers, which can be inorganic or organic. Examples of inorganic fillers include, but are not limited to, barytes, chalk, gypsum, kieserite, sodium carbonate, titanium dioxide, cerium oxide, silica dioxide, kaolin, carbon black, and hollow glass microbeads. Examples of organic fillers include, but are not limited to, powders based on polystyrene, polyvinyl chloride, urea-formaldehyde, and polyethylene. The fillers may be in the form of fibers, such as chopped fibers. Examples of suitable chopped fibers include glass fibers (typically 0.1 millimeter (mm) to 1 mm long) or fibers of organic origin such as, for example, polyester or polyamide fibers.

In order to confer color to the polymer compositions it is possible to use dyes or colored pigments of an organic or inorganic basis such as, for example, iron oxide or chromium oxide pigments or phthalocyanine- or monoazo-based pigments.

METHODS OF PREPARATION OF POLYMER COMPOSITIONS AND ARTICLES

Whether, starting with monomers and polymerizing the monomers in the presence of the bioactive agent, or adding a bioactive agent to a previously prepared polymer, the components are combined in a manner to produce a polymer composition having a bioactive agent dispersed therein.

For certain embodiments, the components are combined in a manner to produce a polymer composition wherein at least a portion of the bioactive agent is incorporated within microparticles. Preferably, this results from combining the components in the presence of water (e.g., 5-10 wt-%, based on the total weight of the composition) and then optionally removing a substantial portion of the water (such that less than 1 wt-% water is remaining, based on the total weight of the composition). If desired, all the water can be removed.

In certain embodiments, an inverse emulsion that includes hydrophilic organic microparticles is combined with water and a bioactive agent under conditions effective to distribute (preferably, dissolve) at least a portion of the bioactive agent in the hydrophilic organic microparticles. Optionally, a secondary organic polymer and/or a foaming agent can be added to the mixture of the inverse emulsion, water, and bioactive agent. Once sufficiently mixed to impregnate at least a portion of the bioactive agent (e.g., silver compound) into the hydrophilic particles, the water is removed if desired.

In other embodiments, monomers for a hydrophilic organic polymer are combined with a bioactive agent, and optionally a foaming agent, under conditions effective to polymerize the monomers and distribute (preferably dissolve) at least a portion of the bioactive agent in the hydrophilic organic polymer. The bioactive agent can be present during the polymerization process or added after the polymerization is complete. Optionally, a secondary organic polymer and/or a foaming agent can be added to the hydrophilic organic polymer with the bioactive agent distributed therein.

The polymer compositions with the bioactive agent therein can be melt processed (e.g., extruded or molded) or solvent cast to form the desired products (e.g., wound dressing). If thermally expandable microspheres (or other foaming agents) are present, the composition can be processed under conditions effective to expand the thermally

expandable microspheres (or other foaming agents) *in situ* during the extrusion process, or after extrusion of the composition followed by exposure to heat in an oven. Thus, in certain embodiments a method of the present invention includes processing the composition under conditions that do not significantly expand the thermally expandable microspheres and subsequently exposing the extruded material to conditions effective to expand the thermally expandable microspheres.

The materials used to prepare the polymer compositions of the present invention are melt processable if they are fluid or pumpable, and they do not significantly degrade or gel at the temperatures used to melt process (e.g., extruding or compounding) the composition (e.g., at least 50°C and up to 300°C). Preferably, such materials have a melt viscosity of at least 10 poise and often up to 1,000,000 poise, as measured by capillary melt rheometry at the processing temperatures and shear rates employed in extrusion. Typically, suitable materials possess a melt viscosity within this range at a temperature of at least 175°C and often up to 225°C and a shear rate of 100 seconds$^{-1}$.

Continuous melt process forming methods include drawing the extruded composition out of a film die and subsequently contacting a moving plastic web or other suitable backing. Another continuous forming method involves directly contacting the extruded composition to a rapidly moving plastic web or other suitable substrate. In this method, the extruded composition can be applied to a moving web using a die having flexible die lips such a reverse orifice coating die and other contact dies using rotating rods. The composition can also be extruded in the form of continuous fibers and blown micro-fiber webs as disclosed in Wente, Van A., "Superfine Thermoplastic Fibers," Industrial Engineering Chemistry, Vol. 48, pp. 1342-1346; Wente, Van A. et al., "Manufacture of Superfine Organic Fibers," Report No. 4364 of the Naval Research Laboratories, published May 25, 1954; U.S. Pat. No. 5,176,952 and U.S. Pat. No. 3,841,953. After melt process forming the composition is solidified by quenching using either direct methods, such as chill rolls or water baths, or indirect methods, such as air or gas impingement, or both.

In some embodiments, a non-adherent or adherent composition (which can be in the form of a gel) is preferably obtained by hot mixing without a solvent (so-called hotmelt process), by blending an elastomer with an oily plasticizer and antioxidants, and then by adding a hydrocolloid either as finely divided powder or as an inverse emulsion. If active agents are provided, these may be added to either the elastomer or the hydrocolloid.

Articles can be prepared using compositions described herein according to a variety of methods, particularly coating methods. When a porous substrate is coated, the process of coating the porous substrate with the composition typically allows the yarns, filaments, or film to be properly trapped in the composition, while leaving most of the apertures unobstructed by the composition. Depending on the structure of the support used, the amount of composition employed will vary over a wide range (typically from 50 grams per square meter (g/m$^2$) to 300 g/m$^2$, and preferably from 60 g/m$^2$ to 160 g/m$^2$).

In certain embodiments, the coating can be carried out hot, without a solvent, using a continuous process in which the substrate is directed over a first coating roll covered with a layer of molten composition having a predetermined thickness, and then over a second roll which removes the composition lying within the apertures of the substrate. The substrate thus covered with gel only on the yarns, filaments, or film is then cooled in a stream of air so that the composition cannot flow and remains uniformly distributed around the yarns, filaments, or film. If necessary, a system producing a laminar stream of air is provided, which system is able both to correct the distribution of the composition around the yarns, filaments, or film and to unblock any substrate apertures, which would not have been open in the previous step of the process.

According to a variant of this process, a substrate can be passed through a bath of molten polymeric composition (for example, at a temperature of 120°C to 200°C). The substrate covered with molten composition is then passed between two fixed rolls pressed against each other with a predetermined gap, so as to remove the excess composition. The amount of composition remaining on the yarns, filaments, or film depends essentially on the gap set between the fixed rolls. The covered process is then cooled and treated in a manner similar to the previous process.

If desired, the cooled coated substrate can be covered with two protective films (for example, thin polyester films). These films may or may not require a nonstick treatment and can function to facilitate extraction from a package and in handling the article. If desired, the coated substrate can be cut into individual compresses, of sizes suitable for the use, packaged in sealed sachets, and sterilized.

Solvent casting may also be used to prepare the articles of the present invention. This method typically employs a common solvent, selected for compatibility with the polymer composition components. Such common solvents include, for example, toluene and tetrahydrofuran. Specific selection of a common solvent for a particular subset of the present invention is within the skill of the art. In the solvent casting method, the materials included in the composition are blended to form a uniform mixture, then coated onto a carrier web or a backing (described below) using a known coating technique such as curtain coating, die coating, knife coating, roll coating, or spray coating. A preferred coating method is knife coating. The solvent is then removed from the coated backing, usually with the aid of a drying oven for a time and temperature selected to remove any undesirable level of residual solvent.

Layered constructions can also be prepared using lamination, coating, or extrusion techniques known to one of skill in the art and as described, for example, in U.S. Pat. No: 6,379,791.

If desired, compositions of the present invention can be sterilized. Methods of sterilization include treatment with electron beam or gamma radiation.

MEDICAL ARTICLES

The polymer compositions of the present invention can be used in a wide variety of products, although they are preferably used in medical articles. Such medical articles can be in the form of a wound dressing, wound packing material, or other material that is applied directly to or contacts a wound.

Such articles may or may not include a backing (i.e., a support substrate). If a backing or support substrate is desired, it can be porous or nonporous. The composition of the present invention can be coated on the support substrate or impregnated into it, for example.

Suitable materials are preferably flexible, and may be fabric, non-woven or woven polymeric films, metallic foils, paper, and/or combinations thereof. More specifically, film backings are useful with the polymer compositions of the present invention. For certain embodiments it is desirable to use a permeable (e.g., with respect to moisture vapor), open apertured substrate (i.e., a scrim). For certain embodiments it is desirable to use an open- or closed-cell foam, such as that disclosed in U.S. Patent Nos. 6,548,727 and 5,409,472.

The porous substrates (i.e., backings) are preferably porous to allow the passage of wound fluids, moisture vapor, and air. In certain embodiments, the porous substrates are substantially impervious to liquid, especially wound exudate. In certain embodiments, the porous substrates are capable of absorbing liquid, especially wound exudate. In certain embodiments, the porous substrate is an apertured, liquid permeable substrate.

Suitable porous substrates include knits, wovens (e.g., cheese cloth and gauze), nonwovens (including spun-bonded nonwovens), extruded porous sheets, and perforated sheets. The apertures (i.e., openings) in the porous substrates are of sufficient size and sufficient number to facilitate high breathability. For certain embodiments, the porous substrates have at least 1 aperture per square centimeter. For certain embodiments, the porous substrates have no greater than 225 apertures per square centimeter. For certain embodiments, the apertures have an average opening size (i.e., the largest dimension of the opening) of at least 0.1 millimeter (mm). For certain embodiments, the apertures have an average opening size (i.e., the largest dimension of the opening) of no greater than 0.5 cm.

For certain embodiments, the porous substrates have a basis weight of at least 5 grams/meter$^2$. For certain embodiments, the porous substrates have a basis weight of no greater than 200 grams/meter$^2$.

The porous substrates (i.e., backings) are preferably flexible yet resistant to tearing. For certain embodiments, the thickness of the porous substrates is at least 0.0125 mm. For certain embodiments, the thickness of the porous substrates is no greater than 3 mm.

The porous substrates may be opaque or translucent. Normally they have a skin color, but "designer" colors and patterns, as well as cartoon character designs, are becoming popular.

Materials of the backing or support substrate include a wide variety of materials including paper, natural or synthetic fibers, threads and yarns made from materials such as cotton, rayon, wool, hemp, jute, nylon, polyesters, polyacetates, polyacrylics, alginates, ethylene-propylene-diene rubbers, natural rubber, polyesters, polyisobutylenes, polyolefins (e.g., polypropylene polyethylene, ethylene propylene copolymers, and ethylene butylene copolymers), polyurethanes (including polyurethane foams), vinyls including polyvinylchloride and ethylene-vinyl acetate, polyamides, polystyrenes, fiberglass, ceramic fibers, and/or combinations thereof.

The backing can also be provided with stretch-release properties. Stretch-release refers to the property of an adhesive article characterized in that, when the article is pulled from a surface, the article detaches from the surface without leaving significant visible residue. For example, a film backing can be formed from a highly extensible and highly elastic composition that includes elastomeric and thermoplastic A-B-A block copolymers, having a low rubber modulus, a lengthwise elongation to break of at least 200%, and a 50% rubber modulus of not above 2,000 pounds/square inch (13.8 megapascals (MPa)). Such backings are described in U.S. Pat. No. 4,024,312 (Korpman). Alternatively, the backing can be highly extensible and substantially non-recoverable such as those described in U.S. Pat. No. 5,516,581 (Kreckel et al,).

Pressure sensitive adhesives used in medical articles can be used in articles of the present invention. That is, a pressure sensitive adhesive material could be applied to the article of this invention, for example, around the periphery, to adhere the article to the skin.

In another aspect, the compositions of the present invention will be in the form of an aqueous gel. Suitable gelling agents include polyoxyethylene-polyoxypropylene diol block copolymers, polyacrylic acid lightly crosslinked with triallyl sucrose which has been neutralised using an alkali metal hydroxide, cellulosic derivatives such as carboxymethyl cellulose, hydroxymethyl cellulose, natural gums, and the like. It will be appreciated that care must be taken to avoid using gelling agents that are incompatible with that bioactive agent, such as silver ions. Suitable gel forming block copolymers of polyoxyethylene- polyoxypropylene will have a molecular weight from 4,600 to 13,500 (approximately) and will be present in the gel in an amount from 50% for the lower molecular weight copolymers to 20% for the higher

molecular weight copolymers, so that the gel when applied topically is neither too stiff nor too fluid. Typically the gels are formed by mixing together the copolymer and water to form an aqueous solution at a temperature of 2°C and adding the bioactive agent (e.g., silver compound) and then allowing the solution to gel as it warms to ambient temperature. A preferred group of gelling agents are the polyoxyethylene-polyoxypropylene diol block copolymers which are commercially available under the trade designation PLURONICS from BASF-Wyandotte (e.g., PLURONICS F108, F127, and P105).

EXAMPLES

Objects and advantages of this invention are further illustrated by the following examples, but the particular materials and amounts thereof recited in these examples, as well as other conditions and details, should not be construed to unduly limit this invention.

*Materials*

IRGACURE 2959 - UV photo-initiator, available from Ciba Specialty Chemicals, Tarrytown, New York.

AGEFLEX FAIQ80MC - 2-(dimethylamino)ethylacrylate methyl chloride quaternary salt (80 wt-% in water) available from Ciba Specialty Chemicals, Tarrytown, New York.

KRATON D1107 - styrene-isoprene-styrene thermoplastic elastomer available from Kraton Polymers, Houston, Texas.

KRATON D4433 - a pre-compounded KRATON D1112 and mineral oil (77/23) blend, where the KRATON D1112P is a linear polystyrene-polyisoprene-polystyrene (SIS) thermoplastic elastomeric copolymer having 15 wt-% polystyrene. The blend is available from Kraton Polymers, Houston, Texas.

KRATON D1124K - radial 4-arm star polystyrene-polyisoprene $(SI)_4$ thermoplastic elastomeric copolymer having 30 wt-% polystyrene available from Kraton Polymers, Houston, Texas.

KAYDOL - mineral oil available from Crompton Corporation, formerly Witco Corporation.

ESCOREZ 1310LC - aliphatic C5 tackifying resin compatible with isoprene block of KRATON D1107 available from Exxon Chemical Company.

IRGANOX 1010 - antioxidant available from Ciba Specialty Chemicals, Tarrytown, New York.

SALCARE SC91 - 50 wt-% solids cosmetic grade emulsion having microparticles of chemically crosslinked hydrophilic anionic sodium acrylates copolymer in mineral and paraffin oils available from Ciba Specialty Chemicals, High Point, North Carolina.

SALCARE SC95 - 50 wt-% solids cosmetic grade emulsion having microparticles of chemically crosslinked hydrophilic cationic quaternary ammonium acrylate polymer (methylchloride quaternary ammonium salt of DMAEMA) in mineral and paraffin oils available from Ciba Specialty Chemicals, High Point, North Carolina.

SALCARE SC96 - 50 wt-% solids cosmetic grade emulsion having microparticles of chemically crosslinked hydrophilic cationic quaternary ammonium acrylate polymer (methylchloride quaternary ammonium salt of DMAEMA) in propylene glycol dicaprylate dicaprate available from Ciba Specialty Chemicals, High Point, North Carolina.

DMAEMA - 2-(dimethylamino)ethyl methacrylate polymer.

Silver Nitrate ($AgNO_3$) - 99+% reagent grade; Formula Weight (FW) is 169.88 from Aldrich (Milwaukee, Wisconsin) used to make a 5.6M $AgNO_3$ solution by dissolving the as received $AgNO_3$ in water..

MICROPEARL F100D - thermally expandable micro-sphere physical foaming agent available from Pierce and Stevens,Buffalo, New York.

Trypticase (Tryptic) Soy Broth (TSB) medium available from Becton Dickinson & Company, Bedford, Massachusetts.

Polyester Knitted Fabric was a 24 mesh polyester knit (61g/$m^2$) purchased from Lamports Filter Media, Inc, Cleveland, OH.

Absorbency Tests

*Bovine Serum Absorbency Test*

A dry wound dressing sample (10 cm x 15 cm) was applied to the upper flange of a clear polycarbonate cup, similar to a Paddington cup as described in the British Pharmacopoeia, 1993, Addendum 1996, page 1943, HMSO London, England. The sample was positioned over the center of the cup cavity (3.8-centimeter (cm) diameter, 3-cm depth, 14-mL volume capacity) and the sample was held in place by its own pressure sensitive adhesive layer. The cup was then inverted and 12 grams (g) of calf bovine serum (Sigma-Aldrich Chemical Co.) was added to the cup through a port. The port was closed with a threaded plug and the cup was placed in an incubator at 40°C and 20% RH. After 24,

48, and 72 hours the amount of unabsorbed serum was removed, weighed ($W_t$), and then added back into the cup. The cup plus sample were then returned to the incubator until the next sampling timepoint. The absorbency was calculated using the following formula and the results reported in grams as an average of three replications:

$$\text{Calf Bovine Serum Absorbency (g)} = 12\text{ g} - W_t$$

*Saline Absorbency Test*

Samples (2.54 cm by 2.54 cm) were soaked in saline. The samples were removed from the saline at various times and were lightly dabbed with a paper towel. The weight was recorded and the samples were placed back into the saline solution. The weight of saline absorbed per weight of dry coating was calculated as a function of swelling time in the saline using the following equation: (weight saline absorbed)/(dry coating sample weight) = [(saline swollen weight) - (dry sample weight)]/[(dry sample weight) - (weight of substrate)].

Anti-microbial Performance Tests

*2 Hours % Live Bacteria Test*

The effectiveness of a sample was tested using a L-7012, Bacterial Viability Kit, available from Molecular Probes (Eugene, Oregon). The procedure is outlined below using the red, propidium iodide dye, and green, SYTO 9 dye, contained in the kit to stain the live and dead bacteria.

Preparation of bacteria solution: Staphylococcus aureus bacteria were grown in Trypticase (Tryptic) Soy Broth (TSB) medium overnight. Bacteria were concentrated by centrifugation at 10,000 x gravity for 15 minutes (min). Supernatant was removed and the pellet was re-suspended in MilliQ water (filtered through a 0.2 μm pore-size filter) or in Butterfield phosphate buffer (from Hardy Diagnostics, Santa Maria, California). Bacteria solution was diluted to the desired bacteria concentration ($10^7$ cells/milliliters) by measuring the optical density (OD) at 670 nm. For a control experiment, the bacteria solution was incubated with 70% isopropyl alcohol at room temperature for 1 hour (hr) to measure the killed bacteria control. Different volume of live and dead bacteria solutions were mixed to generate a range of percent live solution for calibration purposes.

Sample preparation: All prototypes were prepared by punching out a 1-inch (2.54-cm) diameter samples using a stainless steel punch; sometimes as indicated in the examples a 1-inch (2.54 cm) disk was further cut with scissors in eighths and then evaluated. The amount of sample was weighed, and then transferred to 50 milliliters (mL) sterile conical tubes.

Bacteria labeling and Anti-microbial testing: 7 mL of bacteria solution at initial concentration of approximately $1\times10^8$ bacteria/mL were pipetted into a 50 mL conical tube containing the sample. At the specified time (e.g., 2 hours (hr)), 50 microliter (μL) of the supernatant was pipetted into fluorescent measurement tube which already contained 450 μL of MiliQ water and premixed green dye and red dye solution (1.5 μL dye mixture for 500 μL bacteria solution) was added and the mixture was incubated for 15 minutes in the dark at room temperature. These solutions were then measured by flow cytometry. Cell viability was measured using the BD FACSCaliber flow cytometer (made by Becton Dickinson & Company, Franklin Lakes, New Jersey). The flow cytometer is equipped with an argon-ion laser at 488 nanometers (nm) and 15 milliWatts (mW) output. Data acquisition and analysis were controlled using CellQuest software and PBPAC hardware interface. The light path contained a 488/10 nm blocking filter, then a 530/30 nm filter before the green PMT and a 585/42 nm long pass filter before the red PMT. The sampling rate was around 3000-7000 particles/second. The sheath fluid was FACSFlow by Becton Dickinson. The instrument voltage was 5.5 Volt.

The live cell and dead bacteria responses were established with the 100 % live cell and 100% dead cell (for killed bacteria, bacteria solution was incubated with 70% isopropyl alcohol at room temperature for 1 hr) samples. Different volumes of live and dead bacteria solutions were mixed to generate a range of percent live solutions for calibration purposes. The sample results for bacteria killing ability were interpolated from the standard curve generated from calibration samples. Total bacteria concentration was determined by the measuring of the OD at 670 nm of the bacteria solution.

*Zone of Inhibition Test*

Anti-microbial performance was measured using a Zone of Inhibition test (ZOI) that was performed by the following method. Mueller-Hinton agar was prepared, sterilized and tempered in a water bath at 48-50°C. A suspension of bacteria in sterile phosphate-buffered water was prepared with approximately $10^8$ CFU/ml. The agar was cooled to

48-50°C, inoculated with the bacterial suspension to an approximate concentration of $10^5$ CFU/ml (1:1000). The inoculated agar was swirled to mix and pipetted (approximately 14 ml) into sterile Petri dishes (15 x 100 mm). The seeded agar was allowed to set for about 20 minutes to harden. An alcohol-disinfected die and cutting board were used to cut textile samples to desired size. Sterile forceps were used to place the samples onto the seeded, hardened agar in center of plate. The plate was then placed into an incubator at 35-37°C for overnight (16-24 hours) incubation. After incubation the clear zones, no visible colonies formed, were measured in millimeters (mm) with calipers.

The zone of inhibition (ZOI) is then calculated by the following equation:

$$\text{ZOI} = [\text{diameter of clear zone (mm)} - \text{diameter of sample (mm)}]/2.$$

*Peel Adhesion Test*

Peel adhesion is measured as 180° peel from steel plates, at 23°C, 50% relative humidity (RH), 305 millimeters per minute (mm/min), 25mm wide using a Model 3M90 Slip/Peel tester (IMASS, Inc., Accord, MA). The samples were conditioned for 24 hours at controlled temperature and humidity. After conditioning the samples were adhered to a stainless steel panel using 2 kilogram (kg) roller and 4 passes. The samples were peeled from the stainless steel plate after 15 minutes of dwell time using a 0.305 meter/minute (m/min) peel rate. Typically two 0.13 meter (m) long samples were measured and the average peel force recorded in ounces/inch (oz/in) and converted to Newtons per decimeter (N/dm).

Example 1

A solution of 18.2 grams (g) 2-(dimethylamino)ethylacrylate methyl chloride quaternary salt (80% in water; AGEFLEX FAIQ80MC), 0.04 g of IRGACURE 2959, 1.61 g of 2M (2 molar) NaCl aqueous solution and 0.12 g polyethylene glycol 600 diacrylate were added to a glass vial and mixed well. To this mixture was added 0.72 g of 1M $AgNO_3$ aqueous solution and the glass vial was capped. The vial was heated and shaken in a hot water bath until a clear solution was obtained. The solution was placed between clear silicone coated release liners and irradiated with UV light (approximately 3000 millijoules per square centimeter ($mJ/cm^2$)) to produce a clear polymer. Non-stable compositions darkened (black or yellow) during UV irradiation. A 1-inch (25.4-millimeter (mm)) diameter disk of this material was gamma irradiated and then tested for anti-microbial activity against Staphylococcus aureus bacteria using the 2 Hours % Live Bacteria Test. Test results indicated 73% of the bacteria were alive after 2 hours.

Example 2

A solution of 17.5 g of 2-(dimethylamino)ethylacrylate methyl chloride quaternary salt (80% in water) and 0.04 g of IRGACURE 2959 were mixed together. While this mixture was stirring, 2.5 g of a 1M $AgNO_3$ aqueous solution was added in small aliquots. The glass vial was capped. The vial was heated and shaken in a hot water bath until a clear solution was obtained. The solution was poured into a mould and cured between silicone release liners for 12 minutes under UV lights. The 40 mils (1 mm) thick silver polymer matrix was gamma irradiated and tested for anti-microbial activity against *Staphylococcus aureus* bacteria using the 2 Hours % Live Bacteria Test. A 1-inch (25.4-mm) diameter circle killed all the bacteria within 2 hours. Further, one eighth of a 1-inch (25.4 mm) diameter (0.036 g) circle killed all the bacteria within 2 hours.

Example 3

A solution of 17.5 g of 2-(dimethylamino)ethylacrylate methyl chloride quaternary salt (80% in water) and 0.04 g of IRGACURE 2959 were mixed together. While this mixture was stirring, 2.5 g of a 1M $AgNO_3$ aqueous solution was added in small aliquots, and 1.18 g of deionized (DI) water was then added. The glass vial was heated and shaken in a hot water bath until a clear solution was obtained. The solution was placed between silicone coated release liners and irradiated with UV light (approximately 3000 $mJ/cm^2$) to produce a clear polymer. The silver polymer matrix was clear after polymerization. Adding more water made the silver/monomer solution become cloudy.

Example 4

A solution of 14.5 g of 2-(dimethylamino)ethylacrylate methyl chloride quaternary salt (80% in water) and 0.04 g of IRGACURE 2959 were mixed together in a glass vial. While this mixture was stirring, 2.5 g of a 1M $AgNO_3$ aqueous

solution was added in small aliquots. Three grams (3 g) of 2-hydroxyethylmethacrylate was then added and the glass vial was capped. The vial was heated and shaken under hot water until a clear solution was obtained. The solution was placed between silicone coated release liners and irradiated with UV light (approximately 3000 mJ/cm$^2$) to produce a clear polymer. The 40 mils (1 mm) thick clear silver polymer matrix was gamma irradiated and tested for anti-microbial activity against *Staphylococcus aureus* bacteria using the 2 Hours % Live Bacteria Test. A 1-inch (25.4 mm) diameter (0.036 g) circle killed 48 % of the bacteria within 2 hours.

Example 5

A solution of 11.5 g of 2-(dimethylamino)ethylacrylate methyl chloride quaternary salt (80% in water) and 0.04 gram of IRGACURE 2959 were mixed together. While this mixture was stirring, 2.5 g of a 1M $AgNO_3$ aqueous solution was added in small aliquots. Six grams of 2-hydroxyethylmethacrylate was then added and the solution turned white. The solution was then placed between silicone coated release liners and irradiated with UV light (approximately 3000 mJ/cm$^2$) to produce a black colored polymer. Even though this example falls within the scope of the invention it would not preferred for most uses due to the black color that develops on UV irradiation.

Example 6

An absorbent foamed film that was used to make Example 6 was prepared by gravimetrically feeding KRATON D 1107P thermoplastic elastomer pellets at 53 grams per minute feed rate into the feed throat (barrel 1) of a 30 millimeter (mm) diameter, fully intermeshing and co-rotating twin-screw extruder (Werner Pfleiderer Ask30) having nine barrels and a length to diameter ratio of 27 to 1. A mixture of ESCOREZ 1310LC solid tackifying resin and IRGANOX 1010 anti-oxidant was melted at 350°F (177°C) and injected into barrel 2 at 53 grams per minute feed rate using a Dynisco grid-melter with a discharging Zenith gear pump. SALCARE SC95 inverse-emulsion polymer was injected at room temperature (22°C) and 75.6 grams per minute feed rate into barrel 4 using a Zenith gear pump. MICROPEARL F100D foaming agent was gravimetrically fed into barrel 7 at 4.5 grams per minute flow rate using an auxiliary single-screw conveying device. The temperatures of the twin-screw extruder (TSE) were maintained at full cooling, 300°F (149°C), 400°F (204°C), 300°F (149°C), 240°F (116°C), 225°F (107°C), 225°F (107°C), 250°F (121°C) and 300°F (149°C) for barrel 1 through 9, respectively. The TSE was controlled at 200 revolutions per minute (rpm). The TSE was discharged using a Zenith gear pump into a 6-inch (15.24-centimeter (cm)) wide single-orifice film die using a conveying hose. The hose, pump and die were all maintained at 300°F (149°C). The film die gap was set to 0.040 inch (1.0 mm). The TSE temperature profile was controlled so that the foaming agent would not start expanding until the end of the TSE. Continued expansion was facilitated in both the conveying hose and film die. The foamed composition was extruded onto 2 paper release liners that were contacted to two polished and chromed steel rolls that were maintained at 40°F (4°C) and 0.040 inch (1.0 mm) gap. The chilled rolls were set at 3 feet (0.9 meter) per minute take-away speed to provide a 0.040 inch (1.0 mm) thick foamed film having 0.5 gram per cubic centimeter (g/cc) density at 22°C. The composition of the resulting foam was 34 wt-% KRATON D1107, 33 wt-% ESCOREZ 1310LC, 1 wt-% IRGANOX 1010,29 wt-% SALCARE SC95 and 3 wt-% MICROPEARL F100D.

Example 6 was prepared by soaking this extruded foam in a 0.01N (Normal) silver nitrate solution for 6 hours. The soaked foam was subsequently dried for 24 hours at 175°F (79°C). The silver nitrate containing foam (Example 6) was analyzed for the timed release of silver ion upon re-hydration with saline solution using inductively coupled plasma-mass spectrometry (ICPMS). A 2 cm diameter disc of Example 6 was placed into 20 mL of a 0.8 wt-% saline solution at 38°C (approximately human body temperature). After 24 hours the swelled foam was removed from the solution. One milliliter (1 mL) of the remaining solution was diluted to 10 mL with saline. The swelled disc of Example 6 was then placed in a fresh 20 mL of saline and soaked for another 24 hours. Once again, the disc was removed and the process repeated for one more soaking. In a separate measurement, a fresh disc of Example 6 was placed in 20 mL of fresh saline and the sample was removed after 72 hours. The amount of silver ion that was leached out of the Example 6 foam as it was re-hydrated in the saline solution for each of the four leachates was measured using a Perkin Elmer Elan 6000 ICPMS against silver standard dissolved in a 5 wt-% nitric acid solution. Due to interference by the presence of sodium chloride the amounts of silver ion are lower estimates. Table 2 summarizes the ICPMS silver ion concentration analysis of the silver nitrate containing foam leachates for Example 6.

Table 2

| [Ag+] after 1st 24 hour saline soak (μg/20 mL) | [Ag+] after 2nd 24 hour saline soak (μg/ 20 mL) | [Ag+] after 3rd 24 hour saline soak (μg/ 20 mL) | Cumulative [Ag+] after 3 - 24 hour saline soaks (μg/20 mL) | [Ag+] after single 72 hour saline soak (μg/ 20 mL) |
|---|---|---|---|---|
| > 9.5 | > 9.5 | > 9.5 | > 28.5 | > 9.7 |

This analysis demonstrates that silver ions are continually leached out of Example 6 after 72 hours of re-hydration in saline solution.

Examples 7-8

The foamed film described in Example 6 was impregnated with two concentrations of silver nitrate solutions. Examples 7 and 8 were prepared by using a #30 Meyer bar to coat a 0.003 inch (0.08 mm) thick coating of either 0.01N (Example 7) or 0.1N silver nitrate solution (Example 8) onto the surface of the foam. The coated foams were dried at 150°C for 15 minutes. Example 8 absorbed 185 weight percent (wt-%) saline solution after 24 hours of swelling time.

Example 7 (0.01N silver nitrate coating) and Example 8 (0.1N silver nitrate coating) were analyzed for anti-microbial performance using the 2 Hours % Live Bacteria Test with the modifications as listed. The initial live bacteria concentration was approximately 1 x $10^8$ counts per mL of deionized water. A 2 cm diameter disc of the example was placed in a 5 mL solution of the live bacteria. After 2 hours of contact the percentage of live bacteria left in the solution was measured. Both Examples 7 and 8 provided for 100% kill of all live bacterial counts.

Comparative Example 9 and Examples 10-11

Comparative Example 9 and Examples 10-11 were prepared in the same manner as Example 6 with the following modifications. KRATON D1107 was gravimetrically fed at 35 grams per minute flow rate into the feed throat (barrel 1) of the TSE. A mixture of ESCOREZ 1310LC and IRGANOX 1010 (IRG. 1010) was melted at 350°F (177°C) and injected at 35 grams per minute flow rate into barrel 4. SALCARE SC95 was injected at room temperature at 76 grams per minute flow rate into barrel 5. The foaming agent (MICROPEARL F100D) was gravimetrically fed in the same manner as for Example 6 at 4.5 grams per minute into barrel 7. A 0.1N silver nitrate solution was dripped into barrel 7 using a peristaltic pump at either 10 grams per minute (Example 10) or 19.2 grams per minute (Example 11). For Comparative Example 9, 19.2 grams per minute of deionized water was dripped into barrel 7 instead of the silver nitrate solution.

The temperatures of the twin-screw extruder (TSE) were maintained at full cooling, 250°F (121°C), 375°F (191°C), 300°F (149°C), 255°F (124°C), 215°F (102°C), 215°F (102°C), 180°F (82°C) and 265°F (129°C) for barrel 1 through 9, respectively. The TSE was controlled at 400 revolutions per minute (rpm). The film die gap was set to 0.060 inch (1.5 mm). The foamed compositions were extruded onto 2 paper release liners that were contacted to two polished and chromed steel rolls that were maintained at 40°F (4°C) and 0.060 inch (1.5 mm) gap. The chilled rolls were set at 3 feet (0.9 meter) per minute take-away speed to provide 0.060-inch (1.5-mm) thick foamed films.

Comparative Example 9 and Examples 10-11 were laminated to 3M TEGADERM adhesive film and sterilized using gamma irradiation at 24.7 kilograys (kGy) dosage. The samples were tested for absorption of bovine serum albumin (BSA) using the Bovine Serum Albumen Absorbency Test. Examples 10 and 11 were tested using the modified 2 Hours % Live Bacteria Test in the same manner as described for Examples 7 and 8. Table 3 contains the compositional information and Table 4 contains the BSA absorbency and the 2 hours % live bacteria test results for Comparative Example 9 and Examples 10-11.

Table 3

| Ex | KRATON D1107 (wt-%) | ESCOREZ 1310LC (wt-%) | SALCARE SC95 (wt-%) | MICROPEARL F100D (wt-%) | IRG. 1010 (wt-%) | DI Water (wt-%) | $AgNO_3$ (wt-%) |
|---|---|---|---|---|---|---|---|
| 9 (Comp) | 20.62 | 20.21 | 44.78 | 2.65 | 0.41 | 11.31 | 0 |
| 10 | 21.81 | 21.37 | 47.35 | 2.80 | 0.44 | 6.12 | 0.11 |
| 11 | 20.62 | 20.21 | 44.78 | 2.65 | 0.41 | 11.12 | 0.19 |

Table 4

| Ex | Density (g/cc) | $AgNO_3$ (wt-%) | Initial Weight (grams) | 24 Hr. BSA Absorb. (wt-%) | 48 Hr. BSA Absorb. (wt-%) | 72 Hr. BSA Absorb. (wt-%) | 2 Hours % Live Bacteria |
|---|---|---|---|---|---|---|---|
| 9 (Comp) | 0.56 | 0 | 0.57 | 647 | 937 | 1172 | 55.1 |
| 10 | 0.72 | 0.11 | 0.65 | 582 | 865 | 1092 | 32.9 |
| 11 | 0.73 | 0.19 | 0.75 | 483 | 684 | 859 | 6.4 |

Comparative Examples 12,16-18 and Examples 13-15

Fifty (50) grams of deionized (DI) water and 50 grams of silver nitrate (formula weight 169.87) were dissolved to make a 5.89 molar silver nitrate solution. One hundred (100) grams of either SALCARE SC95, SC96, or SC91 were placed in a WARING blender 7012 Model 34BL21 and stirred at the lowest motor setting. Either 1 or 2 milliliters of a 5.89M silver nitrate solution were added drop-wise with a 22 gauge, 1.5-inch (3.75 cm) long stainless steel syringe needle at a rate of 1 drop per second. Once all of the silver nitrate solution had been added, 1 drop of the silver/SALCARE dispersion was placed between two microscope slides and subsequently exposed to 30 minutes of sunlight. Table 5 summarizes the compositions and sunlight stability of Comparative Examples 12,16-18 and Examples 13-15.

Table 5

| Ex | SALCARE SC91 (wt-%) | SALCARE SC95 (wt-%) | SALCARE SC96 (wt-%) | $AgNO_3$ (wt-%) | Did the example darken with sunlight exposure? |
|---|---|---|---|---|---|
| 12 (Comp) | 0 | 0 | 100 | 0 | No |
| 13 | 0 | 0 | 99 | 1 | No |
| 14 | 0 | 0 | 98 | 2 | No |
| 15 | 0 | 98 | 0 | 2 | No |
| 16 (Comp) | 100 | 0 | 0 | 0 | No |
| 17 (Comp) | 99 | 0 | 0 | 1 | Yes |
| 18 (Comp) | 98 | 0 | 0 | 2 | Yes |

The sunlight exposure results presented in Table 5 demonstrate that both the SALCARE SC96 and SC95 mixtures with silver nitrate provided for light stability whereas the presence of SALCARE SC91 did not.

Some of the Examples were tested for anti-microbial activity against *Staph. aureas* using the 2 Hour % Live Bacteria Test. Two drops of the silver/SALCARE dispersion was dripped into the bacterial solution. All bacterial solution volumes were 7 milliliters (mL). The results are tabulated in Table 6. These results can be compared to a standard solution of 0.5 wt-% silver nitrate in DI (containing a calculated $Ag^+$ weight of 22,224 $\mu$g), which demonstrated 15.8% live bacteria after 2 hours.

Table 6

| Example | Sample Weight (grams) | Calc. Silver Salt Weight ($\mu$g) | Calc. $Ag^+$ Weight ($\mu$g) | Initial Live Bacteria Concentration (bacteria/mL) | % Live after 2 hours |
|---|---|---|---|---|---|
| 13 | 0.040 | 400 | 254 | $1.8 \times 10^8$ | 8.2 |
| 14 | 0.040 | 800 | 508 | $1.8 \times 10^8$ | 9.3 |
| 15 | 0.040 | 800 | 508 | $1.8 \times 10^8$ | 38.8 |

Examples 19-21 and Comparative Example 22

Examples 19-21 were prepared in the same manner as Comparative Example 9 and Examples 10-11 except for the following modifications. Two mixtures of SALCARE SC95 emulsion and silver nitrate solutions were prepared by blending a 50 wt-% silver nitrate in deionized water solution into the emulsion using a double planetary Ross mixer. The resulting mixtures consisted of either 98/1/1 or 96/2/2 SALCARE SC95/silver nitrate/deionized water, all in weight percentages. KRATON D1107 was gravimetrically fed into the feed throat (barrel 1) of the TSE. A 98/2 mixture of ESCOREZ 1310LC and IRGANOX 1010 was melted at 350°F (177°C) and injected into barrel 4. The SALCARE SC95/silver nitrate/deionized water mixture was injected at room temperature into barrel 5. The foaming agent (MICROPEARL F100D) was gravimetrically fed in the same manner as for Example 6 into barrel 7 for Examples 10-11.

The temperatures of the twin-screw extruder (TSE) were maintained at full cooling, 300°F (149°C), 400°F (204°C), 300°F (149°C), 240°F (116°C), 225°F (107°C), 225°F (107°C), 250°F (121°C) and 300°F (149°C) for barrel 1 through 9, respectively. The TSE was controlled at 200 revolutions per minute (rpm). The total material throughputs were 151.33 grams per minute and 155.87 grams per minute for Example 19 and Examples 20-21, respectively. The film die gap was set to 0.015 inch (0.25 mm) for Example 19 and 0.060 inch (1.0 mm) for Examples 20-21.

The compositions were extruded onto 2 paper release liners that were contacted to two polished and chromed steel rolls that were maintained at 40°F (4°C) at 0.015 inch (0.25 mm) gap for Example 19 and 0.060 inch (1.5 mm) gap for Examples 20-21. The chilled rolls were set at 3 feet (0.9 meter) per minute take-away speed to provide 0.015-inch (0.25-mm) or 0.060-inch (1.5-mm) thick films for Example 19 and Examples 20-21, respectively. The un-foamed Example 19 had an approximate density of 1.0 gram/cm$^3$ whereas the foamed Examples 20-21 had an approximate density of 0.6 gram/cm$^3$. Table 7 contains the compositional information and for Examples 19-21.

Table 7

| Ex | KRATON D1107 (wt-%) | ESCOREZ 1310LC (wt-%) | SALCARE SC95 (wt-%) | MICROPEARL F100D (wt-%) | IRG. 1010 (wt-%) | DI Water (wt-%) | $AgNO_3$ (wt-%) |
|---|---|---|---|---|---|---|---|
| 19 | 25.00 | 24.00 | 49.00 | 0.00 | 1.00 | 0.50 | 0.50 |
| 20 | 24.27 | 23.30 | 47.58 | 2.91 | 0.97 | 0.49 | 0.49 |
| 21 | 24.27 | 23.30 | 46.61 | 2.91 | 0.97 | 0.97 | 0.97 |

Examples 19-21 and Comparative Example 22 (Contreet H silver hydrocolloid dressing, available from Coloplast Pty. Limited) were evaluated for anti-microbial activity against Staph. aureas using the 2 Hour % Live Bacteria test. All solution volumes were 7 mL. The results are summarized in Table 8.

Table 8

| Example | Sample Weight (grams) | Calc. $AgNO_3$ Weight ($\mu$g) | Calc. Ag+ Weight ($\mu$g) | Initial Live Bacteria Concentration (bacteria/mL) | % Live after 2 hours |
|---|---|---|---|---|---|
| 19 | 0.1247 | 624 | 396 | 1.8 x $10^8$ | 53.1 |
| 20 | 0.0787 | 394 | 250 | 1.8 x $10^8$ | 30.4 |
| 21 | 0.0718 | 718 | 456 | 1.8 x $10^8$ | 28.8 |
| 22 (Comp) | 0.120 | Unknown | Unknown | 1.8 x $10^8$ | 95.5 |

Examples 23-24

Examples 23 and 24 were prepared by first preparing a gel as described below and combining that with a lot of silver modified Salcare that was prepared as outlined below.

*Preparation of Gel*

Two lots of Styrene-isoprene-styrene (SIS) gel were prepared in the following manner. SIS pellets were gravimetrically fed into the feed throat (barrel 1) of a Werner Pfleiderer ZSK30 co-rotating twin-screw extruder (TSE) having

a 30 mm diameter barrel and 15 barrel sections. Each temperature zone was a combination of two barrel sections (e.g., Zone 1 corresponded to barrel sections 2 and 3). Barrel section 1 was controlled at full cooling capacity for all SIS gel lots. A powdered antioxidant (IRGANOX 1010) was also gravimetrically fed into barrel section 1 for SIS gel lot 2. KAYDOL mineral oil was heated and added to the TSE as described in International Publication No. WO 97/00163. The disclosed compounding process provides a method for making a gel by melting of the SIS elastomer followed by addition of the heated mineral oil. Heated mineral oil was sequentially injected into barrel sections 4, 6, 8, 10 and 12, respectively. The TSE screw speed for lots 1-2 was controlled to 400 rpm. The TSE temperature profile for lot 1 was controlled to 204°C, 204°C, 204°C, 191 °C, 177°C, 149°C, and 149°C for zones 1-7, respectively. The heated oil injections for lot 1 were controlled to 204°C, 204°C, 177°C, 149°C, and 149°C, respectively. The TSE temperature profile for lot 2 was controlled to 204°C, 227°C, 227°C, 204°C, 182°C, 171°C, and 93°C for zones 1-7, respectively. The heated oil injections for lot 2 were controlled to 204°C, 204°C, 204°C, 177°C, and 177°C, respectively. Table 9 contains the material flow rates and Table 10 contains the compositional information for SIS gel lots 1-2.

Table 9. SIS Gel Lot Flow Rates

| SIS Gel Lot Number | SIS (g/min) | Barrel Section(S) and Oil addition number and Rate (g/min) | | | | | Total KAYDOL Oil (g/min) | IRGANOX 1010 (g/min) | Total Flow Rate (g/min) |
|---|---|---|---|---|---|---|---|---|---|
| | | S4 Oil 1 | S6 Oil 2 | S8 Oil 3 | S10 Oil 4 | S12 Oil 5 | | | |
| 1 | 125 | 41 | 55 | 40 | 30 | 30 | 196 | - | 321 |
| 2 | 227 | 74 | 100 | 120 | 120 | 108 | 522 | 8 | 757 |

Table 10. SIS Gel Lots 1-2 Compositions

| SIS Gel Lot Number | SIS Type | SIS (wt-%) | KAYDOL oil (wt-%) | IRGANOX 1010 (wt-%) | Total SIS Elastomer (wt-%) |
|---|---|---|---|---|---|
| 1 | linear | 39.0 | 61.0 | - | 30.0 |
| 2 | radial | 30.0 | 69.0 | 1.0 | 30.0 |

*Preparation of the Silver-modified Particles*

Two lots of silver nitrate-modified SALCARE SC95 were prepared. Lot 1 was prepared by mixing 100 grams of SC95 with 2 milliliters (mls) of 5.6 molar (M) silver nitrate at a high speed using a 2-inch (5.08-cm) diameter, three-blade stainless steel paddle mixer. The silver nitrate solution was added drop wise such that all of the solution was added over ten minutes. After all of the silver nitrate solution was added the mixture was further mixed for another ten minutes. Lot 2 was prepared in a similar manner as Lot 1 except twice as much silver nitrate solution was added and the final mixture was dehydrated in a Ross mixer operating at 60°C, 11 hertz and 28 inches (711 mm) of mercury vacuum for 6 hours. Table 11 contains the compositional information for SALCARE SC95/$AgNO_3$ lots 1-2.

Table 11. SALCARE SC95/$AgNO_3$ Lots 1-2 Compositions

| SALCARE SC95 Lot Number | SALCARE SC95 (grams) | SALCARE SC95 (wt-%) | 5.6M $AgNO_3$ (ml) | 5.6M $AgNO_3$ (wt-%) | DI $H_2O$ (wt-%) |
|---|---|---|---|---|---|
| 1 | 100.0 | 96.0 | 2.0 | 2.0 | 2.0 |
| 2 | 100.0 | 96.2 | 4.0 | 3.8 | Dehydrated |

*Preparation of Examples 23-24*

Examples 23-24 were prepared by combining pre-compounded SIS gel lots 1-2 with pre-compounded SALCARE SC95/$AgNO_3$ lots 1-2 in a Haake 25-mm diameter, fully intermeshing counter-rotating TSE. Example 23 was-prepared by re-melting SIS gel lot 1 in a Bonnot extruder operating at 127°C. The molten gel was injected at 22.8 grams per minute into barrel section 1 of the TSE. SALCARE SC95 lot 1 was injected at ambient temperature into barrel section 3 at 15.2 grams per minute using a Zenith gear pump. The TSE was controlled at 300 rpm screw speed and 149°C

temperature. The total material throughput was 38.0 grams per minute for all Examples. The SIS gel/SALCARE blend was discharged out of the TSE into a transport hose using a Zenith gear pump. The transport hose conveyed the molten gel blend to a 0.15meter (m) wide single orifice film die. The transport hose and die were controlled to 157°C and 159°C, respectively. The molten gel blend was extruded into a nip formed by two polished steel rolls gapped at 0.25 mm and controlled to 106°C. A polyester (PET) knitted fabric (Lamports Filter Media, Inc, Cleveland, OH) having 0.8 mm by 0.7 mm (0.56 $mm^2$) rectangular open apertures, 0.20 mm thickness and 0.15 meter (m) width was fed into the nip at 1.4 meters per minute (m/min) speed. As the fabric exited the molten gel blend/nip the article was cooled in air before being wound up with an inserted paper release liner. Upon cooling, a coated fabric having 78 grams/$m^2$ coating weight and 0.75 mm by 0.6 mm (0.45 $mm^2$) rectangular open apertures was obtained. Example 24 was prepared in the same manner only using Gel lot 2 and SALCARE Lot 2. Table 12 contains the process conditions and Table 13 contains the compositional information for Examples 23-24.

Table 12. Example 23-24 Process Conditions

| Ex. | SIS Gel Input (Barrel Section) | SALCARE Input (Barrel Section) | TSE Temp. (°C) | Transport Hose/Die Temp. (°C) | Steel Roll Temp. (°C) | Steel Roll Gap (mm) | Coating Speed (m/min) | Coating Weight (g/$m^2$) |
|---|---|---|---|---|---|---|---|---|
| 23 | 1 | 3 | 149 | 157/159 | 106 | 0.25 | 1.4 | 78 |
| 24 | 2 | 4 | 127 | 127 | 110 | 0.38 | 2.0 | 83 |

Table 13. Example 23-24 compositions

| Ex. | SIS gel Type (Lot Number) | SIS (wt-%) | IRGANOX 1010 (wt-%) | SALCARE SC95 Lot # | SALCARE (wt-%) | KAYDOL oil (wt-%) | $AgNO_3$ (wt-%) | DI $H_2O$ (wt-%) |
|---|---|---|---|---|---|---|---|---|
| 23 | Linear (1) | 18.0 | - | 1 | 38.4 | 42.0 | 0.8 | 0.8 |
| 24 | Radial (2) | 18.0 | 0.6 | 2 | 38.4 | 41.4 | 1.6 | - |

*Testing of Example 24 Adhesion*

Example 24 (the gel coated PET fabric) and slabs (1 mm thick) having the composition of Example 24 were tested for 180° peel adhesion from stainless steel using the peel adhesion test. Measurements of the instantaneous peel force was measured for two 0.13 m long samples and averaged. The 180° peel adhesion from stainless steel was 0.0 N/dm for both the slab and gel coated PET fabric of Example 24. The extremely low 180° peel adhesion demonstrate the inability of the composition and articles of the invention to form a strong adhesive bond. These low values, for the composition and article, are considered to be non-adhesive or non-adherent.

*Testing of Examples 23-24 Absorbency*

Examples 23-24 were tested for their ability to absorb 0.8 wt-% NaCl (saline) as outlined in the Saline Absorbency Test. Table 14 contains the amount of saline absorbed as a function of time.

Table 14. Saline Absorbency vs. Time for Examples 23-24

| Ex. | SIS gel Type (Lot Number) | SIS (wt-%) | SALCARE Type (Lot Number) | 0.5 hour Saline Absorb. | 1 hour Saline Absorb. | 2 hours Saline Absorb. | 6 hours Saline Absorb. | 24 hours Saline Absorb. |
|---|---|---|---|---|---|---|---|---|
| 23 | Linear (1) | 18.0 | SC95 (1) | 0.9 | 1.2 | 1.3 | 2.0 | 2.2 |
| 24 | Radial (2) | 18.0 | SC95 (2) | 4.5 | 4.5 | 4.3 | nm | nm |

The saline absorbency data demonstrates that the composition and article of the invention can absorb an amount of saline that is 1-5 times their dry weight. All samples remained intact after saline exposure, demonstrating the coatings will remain cohesively intact when swollen in a wound bed environment.

Optical micrographs of Example 24 before and after 2 hours of saline exposure were obtained at 2.5x magnification in reflection mode and analyzed for the size of the aperature by measurements of the resulting micrographs. The aperature area was 0.45 $mm^2$ as coated and 0.35 $mm^2$ in the equilibrium saline hydrated state for Example 24. This demonstrates that Example 24 samples still maintain sufficient open area to allow for excess wound fluids to escape the wound bed and yet are substantially absorbent.

*Testing of Examples-Anti-microbial performance*

Example 24 was tested for anti-microbial performance against *Staph. Aureus* using the Zone of Inhibition Test.

Example 24 was sterilized using a cobalt-γ source at both 25 and 40 kilograys (kGy). The samples were tested in the dry state. All samples had a diameter of 24 mm. Table 15 contains the results from the Zone of Inhibition Test for Example 24 at two sterilization exposure levels and a commercially available silver dressing, Example 25 (Comparative-ACTICOAT available from Smith and Nephew, Largo, Florida).

Table 15. Zone of Inhibition Test Results for Example 24

| Ex. | SIS (wt-%) | SALCARE Type (wt-%) | KAYDOL oil (wt-%) | $AgNO_3$ (wt-%) | IRGANOX 1010 | 20 kGy ZOI (mm) | 40 kGy ZOI (mm) | Ave. ZOI (mm) |
|---|---|---|---|---|---|---|---|---|
| 24 | 18.0 | SC95 (38.4) | 41.4 | 1.6 | 0.6 | 3.5 | 3.7 | 3.6 |
| 25 | - | - | - | - | | - | - | 3.3 |

The results in Table 15 demonstrate the anti-microbial efficacy of this invention. The silver containing dressings of Example 24 has higher measured ZOI than the Example 25, the commercially available dressing. The relative amount of total silver in a one square inch portion of dressing is 0.9 milligrams (mg) of $AgNO_3$ (0.6 mg $Ag^+$) in Example 24, calculated from the known material input amounts and coating weight, and 2.9 mg total silver (1.3 mg ammonia soluble silver - the "active" form) for the Example 25 (Wounds 10(6),179-188, 1988 Health Management Publications). Example 24 dressing has significantly less silver, either total or active form and stills performs better in the ZOI test than the comparative sample.

It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

**1.** A polymer composition preparable by a method comprising combining components comprising:

an organic polymer matrix;
an inverse emulsion comprising absorbent hydrophilic microparticles, wherein the microparticles when in a substantially nonhydrated form have an average particle size of 10 microns or less, and wherein the microparticles comprise an amine-containing organic polymer selected from the group consisting of a poly(quaternary amine), a polylactam, a polyamide, and combinations thereof;
a bioactive agent selected from the group consisting of a silver compound, a copper compound, a zinc compound, and combinations thereof, wherein the silver compound has a solubility of at least 0.1 gram per liter in water; and an optional foaming agent;
wherein the components are combined in a manner to produce a polymer composition wherein at least a portion of the bioactive agent is incorporated within the microparticles.

**2.** The polymer composition of claim 1 wherein the microparticles have an average particle size of 1 micron or less when in a substantially nonhydrated form.

**3.** The polymer composition of claim 1 or 2 further comprising secondary absorbent particles having an average particle size of greater than 10 microns when in a substantially nonhydrated form.

**4.** The polymer composition of any of claims 1 to 3 wherein the microparticles are superabsorbent.

5. The polymer composition of any of claims 1 to 4 wherein the organic polymer matrix comprises an elastomeric polymer.

6. The polymer composition of claim 5 wherein the elastomeric polymer is selected from the group consisting of a polyisoprene, a styrene-diene block copolymer, a natural rubber, a polyurethane, a polyether-block-amide, a poly-alpha-olefin, a (C1-C20)acrylic ester of meth(acrylic) acid, an ethylene-octene copolymer, and combinations thereof.

7. The polymer composition of any of claims 1 to 4 wherein the organic polymer matrix comprises a thermoplastic polymer.

8. A polymer composition comprising a hydrophilic amine-containing polymer having a weight average molecular weight of at least 1000 selected from the group consisting of a poly(quaternary amine), a polylactam, a polyamide, and combinations thereof, and a bioactive agent distributed therein, wherein the bioactive agent is a silver compound that has a solubility of at least 0.1 gram per liter in water.

9. A wound dressing comprising an apertured, liquid permeable substrate and the composition of any of claims 1 to 8 wherein the composition is nonadherent.

## Patentansprüche

1. Polymerzusammensetzung, herstellbar durch ein Verfahren, umfassend das Kombinieren von Komponenten, umfassend:

   eine organische Polymermatrix;
   eine inverse Emulsion, umfassend absorbierende hydrophile Mikropartikel, wobei die Mikropartikel, wenn in einer im Wesentlichen nichthydratisierten Form, eine mittlere Partikelgröße von 10 Mikrometer oder weniger aufweisen und wobei die Mikropartikel ein Amin-enthaltendes organisches Polymer umfassen, ausgewählt aus der Gruppe bestehend aus einem Poly(quaternäres Amin), einem Polylactam, einem Polyamid und Kombinationen davon;
   ein bioaktives Mittel, ausgewählt aus der Gruppe bestehend aus einer Silberverbindung, einer Kupferverbindung, einer Zinkverbindung und Kombinationen davon, wobei die Silberverbindung eine Löslichkeit von wenigstens 0,1 Gramm pro Liter in Wasser aufweist; und
   ein optionales Schaummittel;
   wobei die Komponenten auf eine Weise zum Erzeugen einer Polymerzusammensetzung kombiniert werden, wobei wenigstens ein Teil des bioaktiven Mittels in die Mikropartikel eingearbeitet wird.

2. Polymerzusammensetzung gemäß Anspruch 1, wobei die Mikropartikel, wenn in einer im Wesentlichen nichthydratisierten Form, eine mittlere Partikelgröße von 1 Mikrometer oder weniger aufweisen.

3. Polymerzusammensetzung gemäß Anspruch 1 oder 2, ferner umfassend sekundäre absorbierende Partikel, die, wenn in einer im Wesentlichen nichthydratisierten Form, eine mittlere Partikelgröße von mehr als 10 Mikrometer aufweisen.

4. Polymerzusammensetzung gemäß einem der Ansprüche 1 bis 3, wobei die Mikropartikel superabsorbierend sind.

5. Polymerzusammensetzung gemäß einem der Ansprüche 1 bis 4, wobei die organische Polymermatrix ein elastomeres Polymer umfasst.

6. Polymerzusammensetzung gemäß Anspruch 5, wobei das elastomere Polymer ausgewählt ist aus der Gruppe bestehend aus einem Polyisopren, einem Styrol-Dien-Blockcopolymer, einem natürlichen Kautschuk, einem Polyurethan, einem Polyether-Block-Amid, einem Poly-alpha-olefin, einem (Cl-C20)-Acrylester von Meth(acryl)säure, einem Ethylen-Octen-Copolymer und Kombinationen davon.

7. Polymerzusammensetzung gemäß einem der Ansprüche 1 bis 4, wobei die organische Polymermatrix ein thermoplastisches Polymer umfasst.

8. Polymerzusammensetzung, umfassend ein hydrophiles Amin-enthaltendes Polymer mit einem gewichtsgemittelten

Molekulargewicht von wenigstens 1000, ausgewählt aus der Gruppe bestehend aus einem Poly(quaternäres Amin), einem Polylactam, einem Polyamid und Kombinationen davon, und einem darin verteilten bioaktiven Mittel, wobei das bioaktive Mittel eine Silberverbindung ist, die eine Löslichkeit von wenigstens 0,1 Gramm pro Liter in Wasser aufweist.

**9.** Wundverband, umfassend ein mit Öffnungen versehenes, flüssigkeitsdurchlässiges Substrat und die Zusammensetzung gemäß einem der Ansprüche 1 bis 8, wobei die Zusammensetzung nichthaftend ist.

## Revendications

**1.** Composition de polymères pouvant être préparée par un procédé comprenant la combinaison de composants comprenant :

une matrice de polymère organique ;
une émulsion inverse comprenant des microparticules hydrophiles absorbantes, les microparticules lorsqu'elles sont sous une forme pratiquement non hydratée ayant une taille moyenne de particule inférieure ou égale à 10 micromètres et les microparticules comprenant un polymère organique contenant des groupes amine choisi parmi l'ensemble consistant en une poly(amine quaternaire), un polylactame, un polyamide et des associations de ceux-ci ;
un agent bioactif choisi parmi l'ensemble consistant en un composé de l'argent, un composé du cuivre, un composé du zinc et des associations de ceux-ci, le composé de l'argent ayant une solubilité d'au moins 0,1 gramme par litre dans l' eau ; et
un agent moussant facultatif ;

dans laquelle les composants sont combinés de manière à produire une composition de polymères dans laquelle au moins une partie de l'agent bioactif est incorporée dans les microparticules.

**2.** Composition de polymères selon la revendication 1 dans laquelle les microparticules ont une taille moyenne de particule inférieure ou égale à 1 micromètre lorsqu'elles sont sous une forme pratiquement non hydratée.

**3.** Composition de polymères selon la revendication 1 ou 2 comprenant en outre des particules absorbantes secondaires ayant une taille moyenne de particule supérieure à 10 micromètres lorsqu'elles sont sous une forme pratiquement non hydratée.

**4.** Composition de polymères selon l'une quelconque des revendications 1 à 3 dans laquelle les microparticules sont superabsorbantes.

**5.** Composition de polymères selon l'une quelconque des revendications 1 à 4 dans laquelle la matrice de polymère organique comprend un polymère élastomère.

**6.** Composition de polymères selon la revendication 5 dans laquelle le polymère élastomère est choisi parmi l'ensemble consistant en un polyisoprène, un copolymère séquencé de styrène-diène, un caoutchouc naturel, un polyuréthane, un polyéther-bloc-amide, une poly(alpha-oléfine), un ester acrylique en C1-C20 de l'acide (méth)acrylique, un copolymère d'éthylène-octène et des associations de ceux-ci.

**7.** Composition de polymères selon l'une quelconque des revendications 1 à 4 dans laquelle la matrice de polymère organique comprend un polymère thermoplastique.

**8.** Composition de polymères comprenant un polymère contenant des groupes amine hydrophile ayant une masse moléculaire moyenne en poids d'au moins 1 000 choisi parmi l'ensemble consistant en une poly(amine quaternaire), un polylactame, un polyamide, et des associations de ceux-ci, et un agent bioactif réparti dans celui-ci, dans laquelle l'agent bioactif est un composé de l'argent qui a une solubilité d'au moins 0,1 gramme par litre dans l'eau.

**9.** Pansement comprenant un substrat troué perméable aux liquides et la composition selon l'une quelconque des revendications 1 à 8 dans lequel la composition est non adhésive.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0489967 A1
- US 6039940 A
- US 5437932 A, Ali
- EP 172724 A2
- EP 126528 A2
- EP 0126528 A2
- WO 9723577 A
- WO 9625469 A
- US 5369155 A
- WO 0074916 A
- US 6103152 A
- US 5476712 A
- US 6284362 B
- US 10387263 B
- US 2006173087 A
- US 5270358 A
- US 5176952 A
- US 3841953 A
- US 6379791 B
- US 6548727 B
- US 5409472 A
- US 4024312 A, Korpman
- US 5516581 A, Kreckel
- WO 9700163 A


### Non-patent literature cited in the description


- **CALVERT ; PITTS.** Photochemistry. John Wiley and Sons, 1966
- *Progress in Organic Coatings,* 1985, vol. 13, 123-150
- **WENTE, VAN A.** Superfine Thermoplastic Fibers. *Industrial Engineering Chemistry,* vol. 48, 1342-1346
- **WENTE, VAN A. et al.** Manufacture of Superfine Organic Fibers. Naval Research Laboratories, 25 May 1954
- *Wounds,* 1988, vol. 10 (6), 179-188